# EUROPÄISCHE PATENTANMELDUNG

(11) **EP 1 010 395 A1**
(43) Veröffentlichungstag der Anmeldung: **21.06.2000**
(21) Anmeldenummer: 99123635.7
(22) Anmeldetag: 27.11.1999
(51) Int. Cl.: A61B 17/00

(54) **Endoskopisches Instrument mit Kopplungseinrichtung**

(30) Priorität: 16.12.1998 AT 83598
(71) Anmelder: AMI (Agency for medical innovations GmbH), 6840 GötzisAT (AT)
(72) Erfinder: Dipl.- Ing. Siegfried Höfle, A-6840 Götzis (AT)
(74) Vertreter: Riebling, Peter, Dr.-Ing.

(57) **Zusammenfassung**

Die Erfindung betrifft ein endoskopisches Instrument mit Kopplungseinrichtung, wobei der endoskopische Applikator aus einem Handgriff besteht, der fest mit einem Applikationsrohr verbunden ist, und in dem Applikationsrohr ein Zugstab axial verschiebbar und abdichtend geführt ist. Auf dem Außenumfang des Applikationsrohres ist eine kürzere Hülse verschiebbar angeordnet und das vordere, distale Ende des Applikationsrohres bildet eine Aufnahmeöffnung, in welche unterschiedliche chirurgische Applikatoren lösbar durch das Einrasten einer Kunststoffrastfeder in eine Arretieröffnung des Applikationsrohres aufgenommen werden können. Der Zugstab weist an seinem distalen Ende einen Haken auf, welcher mit entsprechenden Aufnahmeeinrichtungen am chirurgischen Applikator koppelbar ausgebildet ist. Das Applikationsrohr mit Handgriff, der Zugstab und die Hülse sind voneinander lösbar ausgebildet, so daß eine geeignete Reinigung und ggf. eine Sterilisierung dadurch ermöglicht wird. Eine weitere Ausgestaltung der Erfindung sieht vor, daß der Zugstab schraubenförmig verdrehbar und abdichtend im Applikationsrohr geführt wird.

## Beschreibung

Die Erfindung betrifft ein endoskopisches Instrument mit Kopplungseinrichtung nach dem Oberbegriff der Patentansprüche 1 und 14. Derartige endoskopische Instrumente sind beispielsweise mit dem Gegenstand der DE 34 09 663 C2 oder der US 5 312 333 bekannt geworden. Derartige endoskopische Instrumente dienen zur Ausführung von mikrochirurgischen Operationen, welche mit Hilfe des endoskopischen Instrumentes durch eine entsprechende Körperöffnung hindurch durchgeführt werden.

Bei der US 5 312 333 ist eine Kopplungseinrichtung zwischen einem Handgriff und einem Applikationsrohr der Gestalt so beschrieben, daß das Applikationsrohr in eine Gewindeaufnahme am Handgriff anschraubbar ist und gleichzeitig eine Zugstange vorhanden ist, welche mit dem Betätigungselement im Handgriff koppelbar ausgeführt ist.

Nachteil dieses endoskopischen Applikators ist jedoch, daß die genannte Zugstange nur schwer zu zerlegen ist und im übrigen schwer zu reinigen ist.

Die Kopplungseinrichtung in der Nähe des Handgriffes erfordert, daß der an dem vorderen Ende des Applikationsrohrs angesetzte Applikator zusammen mit dem Rohr ausgewechselt werden muß. Dies ist äußerst nachteilig. Es ist also nicht möglich, auf einem vorhandenen Rohr verschiedene Applikationsinstrumente leicht anzubringen.

Der Erfindung liegt deshalb die Aufgabe zugrunde, einen endoskopischen Applikator der Eingangs genannten Art so weiter zu bilden, daß unterschiedliche endoskopische Instrumente, Applikatoren und Zusatzgeräte leicht koppelbar mit dem endoskopischen Applikator verbunden werden können, leicht abnehmbar und getrennt zu reinigen sind.

Zur Lösung der gestellten Aufgabe dient die technische Lehre nach den Ansprüchen 1 und 14.

Wesentliches Merkmal der Erfindung ist, daß der endoskopische Applikator nun erfindungsgemäß aus einem Handgriff besteht, der fest mit einem Applikationsrohr verbunden ist, und daß in dem Applikationsrohr eine Zugstange verschiebbar geführt ist. Ferner daß auf dem Außenumfang des Applikationsrohres eine in der Länge kürzere Hülse verschiebbar und abdichtend auf dem Außenumfang des Applikationrohres angeordnet ist, und daß das vordere, distale Ende des Applikationsrohres eine Aufnahmeöffnung bildet, in welche unterschiedliche endoskopische Instrumente, Applikatoren einrastbar aufgenommen sind.

Mit der gegebenen technischen Lehre ergibt sich der wesentliche Vorteil, daß mit einem Applikationsrohr, welches fest mit einem Handgriff verbunden ist, an dessen vorderen Ende eine Aufnahmeöffnung für unterschiedliche endoskopische Instrumente und Applikatoren geschaffen wird, daß es nun erstmals möglich ist, derartige endoskopische Instrumente und Applikatoren als Einmalinstrumente auszuführen, die leicht auswechselbar in der Aufnahmeöffnung des Applikationsrohres gehalten sind.

Hiermit ergibt sich also eine Kopplungseinrichtung, die am distalen Ende des Applikationsrohres angeordnet ist und die eben dazu geeignet ist, abdichtend und fest derartige Applikatoren und Instrumente aufzunehmen.

Es wird hierbei bevorzugt, daß eine lösbare Verbindung der Applikatoren in dem Applikationsrohr über eine lösbare Rastverbindung erfolgt. Zu diesem Zweck weist der Applikator eine Kunststoffrastfeder auf die in eine zugeordnete Arretieröffnung am vorderen Ende des Applikationsrohres einrastbar ist.

Neben der lösbaren Rastverbindung werden jedoch auch noch andere lösbare Verbindungen und Befestigungen in der vorliegenden Erfindung als erfindungswesentlich beansprucht.

Beispielsweise können auch entsprechende Schraubverbindungen verwendet werden, wobei entweder ein Links- oder ein Rechtsgewinde verwendet wird.

In einer weiteren Ausgestaltung sind sogenannte Bajonettsteckverbindungen vorgesehen, welche durch Aufstecken und Drehen ebenfalls gesichert und gelöst werden können.

Ein besonderer Vorteil der Erfindung ergibt sich dadurch, daß im Applikationsrohr noch zusätzlich ein Zugstab längsverschiebbar geführt ist, welcher an seinem vorderen, distalen Ende mit einem Haken versehen ist. Es handelt sich also hierbei um ein Betätigungsinstrument, weil mit Verschiebung des Zugstabes der Haken entsprechend in axialer Richtung im Applikationsrohr verschoben werden kann. Der Haken kann nun mit entsprechenden Aufnahmeeinrichtungen am chirurgischen Applikator dem endoskopischen Instrument oder dergleichen gekoppelt werden und demzufolge damit den Applikator betätigen. Beispielsweise kann der Applikator als Spritze ausgebildet werden und der Pumpenkolben dieser Spritze wird mit dem Haken gekoppelt, wodurch er über einen Schieber, der fest mit dem Zugstab verbunden ist, die Spritze am vorderen Ende des Applikationsrohres betätigt werden kann.

Neben der Längsverschiebung eines Zugstabes im Applikationsrohr ist es in einer anderen Ausgestaltung der Erfindung vorgesehen, daß der Zugstab nicht längsverschiebbar ist, sondern daß er schraubenförmig verdrehbar ist. Auf diese Weise würde der Zugstab sowohl verdrehbar, als auch in Längsrichtung hin und her verschiebbar ausgebildet sein.

In einer dritten Ausgestaltung ist es vorgesehen, daß der Zugstab zwar längsverschiebbar in dem Applikationsrohr geführt ist, daß er aber in seinen Endstellungen im Applikationsrohr (d. h. also im eingezogenen oder im ausgeschobenen Zustand) jeweils um einen bestimmten Winkelgrad von z. B. 90 oder 180 Grad verdrehbar ausgebildet ist.

Besonderer Vorteil der Erfindung ist, daß der gesamte endoskopische Applikator leicht zerlegbar ausgebildet ist, denn der Zugstab kann einfach aus dem Applikationsrohr nach hinten herausgezogen werden und ist demzufolge separat reinigungs- und sterilisierfähig.

Ebenso kann die Hülse mit der zugeordneten Verschraubung, welche axial verschiebbar auf dem Applikationsrohr aufgesetzt ist abgezogen, separat gereinigt und sterilisiert werden.

Auch das Applikationsrohr mit dem fest daran angeordneten Handgriff kann separat gereinigt und sterilisiert werden.

Wichtig bei allen Ausführungsformen ist also, daß auf dem distalen Ende des Applikationsrohres eine entsprechende Aufnahmeöffnung für die lösbare Aufnahme von unterschiedlichen endoskopischen Applikatoren, Instrumenten und Teilen vorgesehen ist, daß diese dort leicht lösbar gehalten sind.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung, offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.

Es zeigen:
- Figur 1:: explosionsartige Darstellung eines endoskopischen Applikators
- Figur 2:: der Applikator nach Fig. 1 im zusammengebauten Zustand
- Figur 3:: einen Schnitt durch die Verschraubung
- Figur 4:: der Applikator nach Fig. 1 in Untenansicht
- Figur 5:: einen Schnitt durch den zusammengebauten Applikator mit zurückgezogenem Zugstab
- Figur 6:: einen Schnitt durch den Applikator mit vorgeschobenem Zugstab
- Figur 7:: die Draufsicht auf den Applikator nach Fig. 6

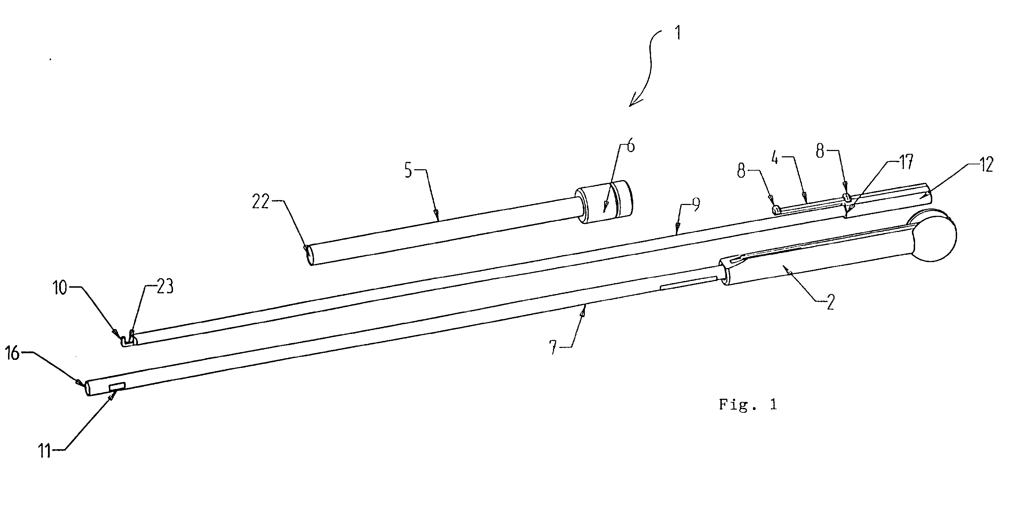

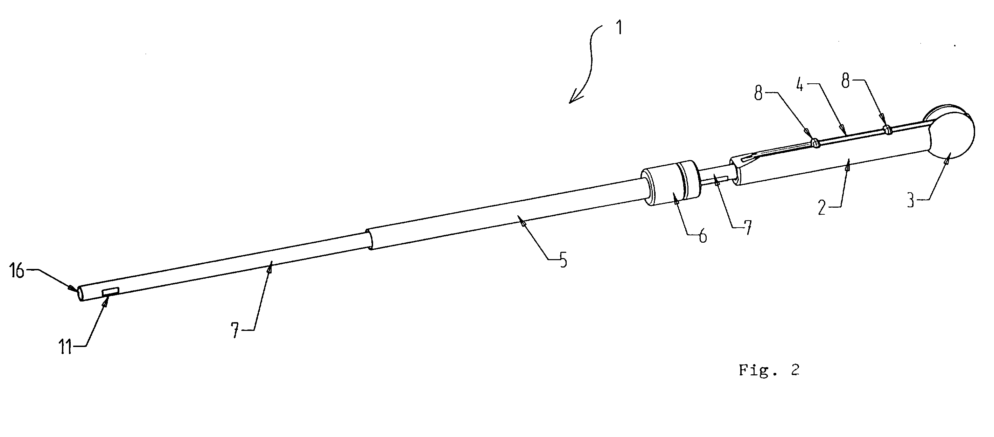

Der endoskopische Applikator 1 nach Fig. 1 und Fig. 2 besteht im wesentlichen aus einem Handgriff 2, der fest mit einem Applikationsrohr 7 verbunden ist. Der Handgriff besteht bevorzugt aus einem Kunststoffmaterial und das Applikationsrohr 7 ist in diesem Handgriff 2 eingepreßt.

Der Handgriff 2 wird nach hinten durch eine Griffkugel 3 abgeschlossen, um eine griffige Ausführung zu gewährleisten.

Im Innenraum des Applikationsrohres ist ein Zugstab 9 axial verschiebbar ausgebildet, welcher Zugstab 9 gemäß Fig. 1 im wesentlichen aus einem länglichen, massiven Stab besteht, an dessen vorderen Ende ein Haken 10 angeformt ist, der nach oben abgebogen ist und demzufolge eine Hakenöffnung 23 definiert. Der Zugstab 9 ist fest mit einem Führungsteil 12 verbunden, welches an seiner Oberseite einen Schieber 4 aufweist, der mit zwei einen gegenseitigen Abstand von einander einnehmenden Nasen 8 ausgebildet ist. Zusätzlich dazu ist am Schieber 4 noch ein nach vorn gerichteter Anschlag 17 angeformt.

Die Länge des Zugstabes 9 ist kürzer gewählt, als die Länge des Applikationsrohres 7, so daß der Zugstab 9 innerhalb des Applikationsrohres 7 vollkommen aufgenommen werden kann und durch Verschiebung in axialer Richtung im Applikationsrohr 7 aus diesem distal teilweise ausgefahren werden kann.

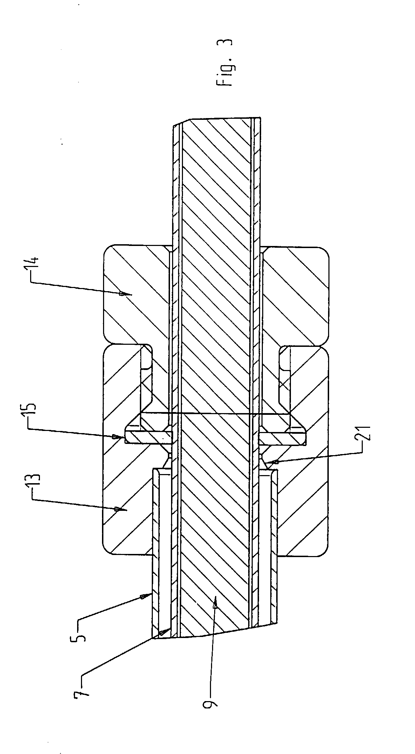

Über das Applikationsrohr 7 wird eine Hülse 5 geschoben, welche ebenfalls rohrförmig ist und die eine vordere Aufnahmeöffnung 22 aufweist. Nach hinten ist diese Hülse 5 durch eine Verschraubung 6 abgeschlossen, wobei die Funktion der Verschraubung sich aus Figur 3 ergibt.

Die Hülse 5 ist hierbei drehfest mit einer Schraubenmutter 13 verbunden, welche die genannten Rohre 7, 9 übergreift und in deren Innenraum ein Dichtungsring 15 angeordnet ist. Dem gegenübergestellt ist eine Schraubhülse 14 vorgesehen, welche in die Schraubenmutter 13 einschraubbar ist und die sich an dem Dichtungsring 15 mit einem stirnseitigen Anschlag anlegt. Auf diese Weise wird der Dichtungsring 15 radial einwärts auf den Außenumfang des Applikationsrohres 7 gepreßt und dichtet diesen Außenumfang ab. Die gesamte Verschraubung 6 ist jedoch in axialer Richtung auf dem Applikationsrohr 7 verschiebbar ausgebildet.

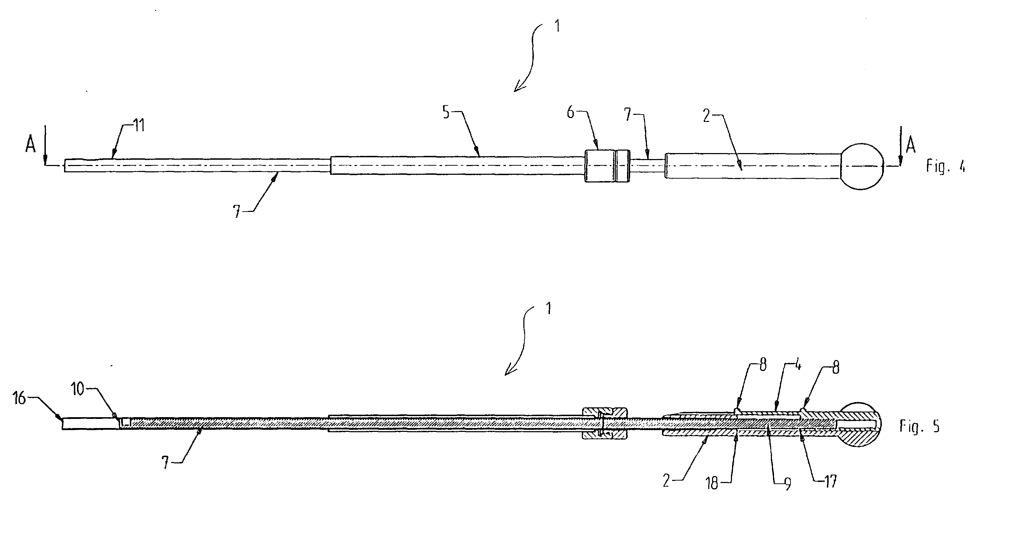

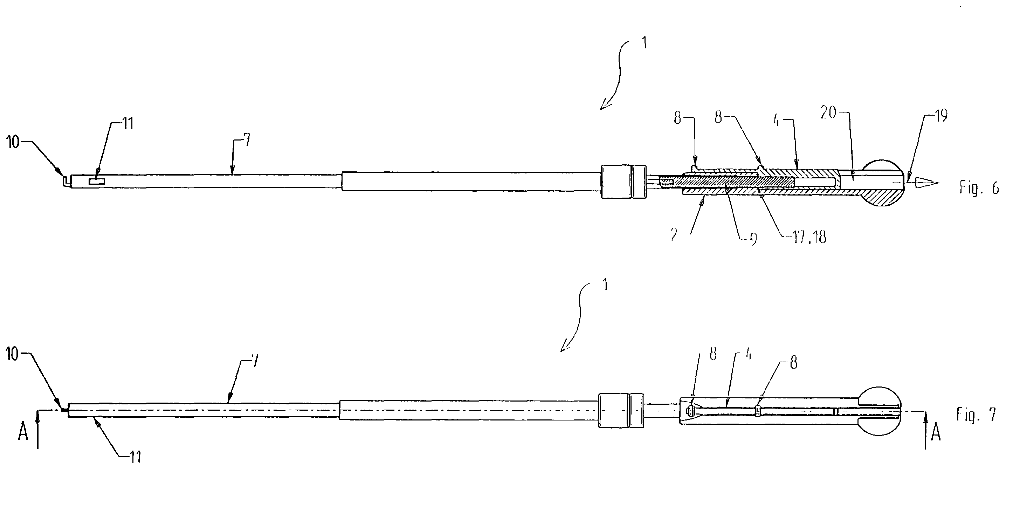

Aus den Figuren 5 und 6 ergeben sich weitere Einzelheiten der Verschiebung des Zugstabes 9. Hierbei ist erkennbar, daß der hintere Anschlag 17 im zurückgezogenen Zustand des Zugstabes 9 nach Fig. 4 keine Funktion hat. Das heißt der Zugstab 9 ist in Pfeilrichtung 19 aus dem Handgriff 2 herausziehbar ausgebildet.

Im vorgeschobenen Zustand des Zugstabes 9 (siehe Figur 6 und 7) greift der Haken aus der vorderen Aufnahmeöffnung 16 des Applikationsrohres 7 heraus und ist geeignet, dort mit einem entsprechenden chirurgischen Instrument oder einem Applikator verbunden zu werden. Im vorgeschobenen Zustand schlägt hierbei der Anschlag 17 des Schiebers 4 mit einem Anschlag 18 im Handgriff 2 zusammen, so daß die vordere Verschiebung des Zugstabes 9 damit begrenzt wird.

Er kann jedoch nach hinten in Pfeilrichtung 19 über die Längsöffnung 20 im Handgriff 2 herausgezogen werden.

Wichtig ist nun, daß die Aufnahmeöffnung 16 eine entsprechende axiale Länge aufweist, so daß in dieser Aufnahmeöffnung verschiedene nicht näher dargestellte zylindrische Ansatzteile, von dort einzusteckenden chirurgischen Instrumenten und Applikatoren dichtend eingesteckt werden können.

Ein derartiger Applikator hat hierbei eine federnde Rastnase, welche dann in Eingriff mit der Arretieröffnung 11 kommt und dort verrastet.

Zum Auswechseln dieses Einmal-Applikators, wird dann einfach die nicht mehr dargestellte Arretiernase in die Arretieröffnung 11 eingedrückt und gleichzeitig wird der Applikator aus der Aufnahmeöffnung 16 herausgezogen.

Damit besteht also der Vorteil, daß unterschiedliche Einmal-Applikatoren sehr schnell und sicher in dem Applikationsrohr 7 verankert werden können, und ebenso schnell entfernt werden können. Es ergeben sich damit wesentliche Kosteneinsparungen, denn der gesamte endoskopische Applikator 1 kann stets wieder neu verwendet werden, weil er sehr leicht zu reinigen und ggf. zu sterilisieren ist, während die, für die chirurgischen Operationen verwendeten endoskopischen Applikatoren als Einmalinstrumente ausgebildet sind und leicht auswechselbar im Applikationsrohr 7 gehalten sind.

Im Betrieb wird zunächst ein endoskopischer Applikator - wie beschrieben - in die Aufnahmeöffnung 16 eingeführt, und in der Arretieröffnung 11 verrastet.

Handelt es sich um einen Applikator indem noch ein längsbewegliches Zusatzelement vorhanden ist, dann kann dieses längsbewegliche Zusatzelement über die Hakenöffnung 23 im Haken 10 mit der Zugstange 9 noch zusätzlich gekoppelt werden.

Der Zugstab 9 wird nun soweit zurückgezogen, daß das vordere, distale Ende des Applikationsrohres 7 in der Aufnahmeöffnung 22 der Hülse 5 aufgenommen ist. Hierbei ist vorteilhaft, ein Anschlagbund 21 geringeren Durchmessers in der Schraubenmutter 13 angeordnet, welcher Anschlagbund einen geringeren Durchmesser hat, als der Innendurchmessers des Applikationsrohres 7. Damit wird das Applikationsrohr 7 mit seinem proximalen Ende an der Schraubenmutter 13 festgelegt. Damit ist für den Operateur die maximal zurückgezogene Stellung des Applikationsrohres 7 definiert.

Die Hülse 5 mit dem dort zurückgezogenen Applikationsrohr 7 und dem gleichfalls in der Hülse verborgenen endoskopischen Instrument wird nun in einen Trocar eingeführt, und dann im Körper entsprechend ausgeschoben, so daß also durch vorschieben des Applikationsrohres 7 in der festgehaltenen Hülse 5 das nicht näher dargestellte chirurgische Instrument in die Nähe des Operationsortes gebracht wird. Es können nun verschiedene Operationen durchgeführt werden, wie z. B. Gewebeentnahme, Flüssigkeitsentnahme und dergleichen mehr, wobei auch der Zugstab 9 betätigt werden kann, um entsprechende, am distalen Ende des Applikationsrohres angeordnete Instrumente zu betätigen.

Nach Durchführung der entsprechenden Tätigkeiten wird das Applikationsrohr 7 wieder in die feststehende Hülse 5 zurückgezogen, so daß auch das endoskopische Instrument in der Hülse 5 aufgenommen ist. Es kann nun der gesamte endoskopische Applikator 1 aus der Körperöffnung (aus dem Trocar) entfernt werden.

### Zeichnungslegende

- 1.: Endoskopischer Applikator
- 2.: Handgriff
- 3.: Griffkugel
- 4.: Schieber
- 5.: Hülse
- 6.: Verschraubung
- 7.: Applikationsrohr
- 8.: Nase
- 9.: Zugstab
- 10.: Haken
- 11.: Arretieröffnung
- 12.: Führungsteil
- 13.: Schraubenmutter
- 14.: Schraubhülse
- 15.: Dichtungsring
- 16.: Aufnahmeöffnung
- 17.: Anschlag (Schieber 4)
- 18.: Anschlag (Handgriff 2)
- 19.: Pfeilrichtung
- 20.: Längsöffnung
- 21.: Anschlagbund
- 22.: Aufnahmeöffnung (Hülse 5)
- 23.: Hakenöffnnung

## Patentansprüche

1. Endoskopisches Instrument mit Kopplungseinrichtung **dadurch gekennzeichnet**, daß der endoskopische Applikator (1) aus einem Handgriff (2) besteht, der fest mit einem Applikationsrohr (7) verbunden ist, und daß in dem Applikationsrohr (7) ein Zugstab (9) axial verschiebbar und abdichtend geführt ist.

2. Endoskopisches Instrument mit Kopplungseinrichtung nach Anspruch 1, **dadurch gekennzeichnet,** daß auf dem Außenumfang des Applikationsrohres (7) eine kürzere Hülse (5) verschiebbar angeordnet ist.

3. Endoskopisches Instrument mit Kopplungseinrichtung nach Anspruch 1 - 2, **dadurch gekennzeichnet,** daß das vordere, distale Ende des Applikationsrohres (7) eine Aufnahmeöffnung (16) bildet, in welche unterschiedliche endoskopische Applikatoren aufgenommen sind.

4. Endoskopisches Instrument mit Kopplungseinrichtung nach Anspruch 3, **dadurch gekennzeichnet,** daß die lösbare Verbindung der Applikatoren in der Aufnahmeöffnung (16) des Applikationsrohres (7) über eine lösbare Rastverbindung erfolgt.

5. Endoskopisches Instrument mit Kopplungseinrichtung nach Anspruch 4, **dadurch gekennzeichnet,** daß die lösbare Verbindung der Applikatoren in der Aufnahmeöffnung (16) des Applikationsrohres (7) über eine Schraubverbindung erfolgt.

6. Endoskopisches Instrument mit Kopplungseinrichtung nach Anspruch 4, **dadurch gekennzeichnet,** daß die lösbare Verbindung der Applikatoren in der Aufnahmeöffnung (16) des Applikationsrohres (7) über eine Bajonettsteckverbindung erfolgt.

7. Endoskopisches Instrument mit Kopplungseinrichtung nach Anspruch 1 - 6, **dadurch gekennzeichnet,** daß der Applikator eine Kunststoffrastfeder aufweist, die in eine zugeordnete Arretieröffnung (11) am vorderen des Applikationsrohres (7) einrastbar ist.

8. Endoskopisches Instrument mit Kopplungseinrichtung nach Anspruch 1 - 7, **dadurch gekennzeichnet,** daß der Zugstab (9) im Applikationsrohr (7) an seinem vorderen, distalen Ende mit einem Haken (10) versehen ist.

9. Endoskopisches Instrument mit Kopplungseinrichtung nach Anspruch 8, **dadurch gekennzeichnet,** daß der Haken (10) mit entsprechenden Aufnahmeeinrichtungen am chirurgischen Applikator koppelbar ausgebildet ist.

10. Endoskopisches Instrument mit Kopplungseinrichtung nach Anspruch 1 - 9, **dadurch gekennzeichnet,** daß bei Koppelung des Applikators als Spritze der Pumpenkolben dieser Spritze mit dem Haken (10) gekoppelt wird, wodurch über einen Schieber (4), der fest mit dem Zugstab (9) verbunden ist, die Spritze am vorderen Ende des Applikationsrohres (7) betätigt wird.

11. Endoskopisches Instrument mit Kopplungseinrichtung nach Anspruch 1 - 10, **dadurch gekennzeichnet,** daß der Zugstab (9) aus dem Applikationsrohr (7) nach hinten vollständig herausgezogen werden kann und vom Applikationsrohr (7) getrennt werden kann.

12. Endoskopisches Instrument mit Kopplungseinrichtung nach Anspruch 1 - 11, **dadurch gekennzeichnet,** daß die axial auf dem Applikationsrohr (7) verschiebbare Hülse (5) zusammen mit ihrer zugeordneten Verschraubung (6) von dem Applikationsrohr (7) abgezogen werden kann.

13. Endoskopisches Instrument mit Kopplungseinrichtung nach Anspruch 1 - 12, **dadurch gekennzeichnet,** daß der Zugstab (9) zusätzlich zu seiner Längsverschiebbarkeit in dem Applikationsrohr (7) in seinen Endstellungen im Applikationsrohr (7) jeweils um einen bestimmten Winkelgrad verdrehbar ausgebildet ist.

14. Endoskopisches Instrument mit Kopplungseinrichtung, **dadurch gekennzeichnet,** daß der endoskopische Applikator (1) aus einem Handgriff (2) besteht, der fest mit einem Applikationsrohr (7) verbunden ist, und daß in dem Applikationsrohr (7) ein Zugstab (9) schraubenförmig verdrehbar ist.

15. Endoskopisches Instrument mit Kopplungseinrichtung nach Anspruch 14, **dadurch gekennzeichnet,** daß der Zugstab (9) in dem Applikationsrohr (7) abdichtend geführt ist.
